# EUROPEAN PATENT APPLICATION

(11) **EP 3 298 965 A1**
(43) Date of publication of application: **28.03.2018**
(21) Application number: 16796205.9
(22) Date of filing: 04.04.2016
(51) Int. Cl.: A61B 8/00

(54) **COATING-TYPE CONTACT MEDIUM FOR ULTRASONIC DIAGNOSIS**

(30) Priority: 20.05.2015 JP 2015102722
(71) Applicant: FUJIFILM Corporation, Tokyo 106-8620 (JP)
(72) Inventor: KUSUNOKI Tetsuro, Ashigarakami-gun Kanagawa 258-8538 (JP)
(74) Representative: Klunker IP Patentanwälte PartG mbB
(86) International application number: PCT/JP2016/061001
(87) International publication number: WO 2016/185811

(57) **Abstract**

The invention provides a coating-type contact medium for ultrasound diagnosis which gives a good feeling when the skin is coated therewith and has antimicrobial effects. The coating-type contact medium for ultrasound diagnosis contains silver ion as a silver-based antimicrobial agent, wherein the silver-based antimicrobial agent is at least one selected from the group consisting of silver oxide, ceramic-supported silver, and glass-supported silver, and at least one compound selected from the group consisting of a viscosity improver and a lubricant.

## Description

### BACKGROUND OF THE INVENTION

### 1. Field of the Invention

The present invention relates to a coating-type contact medium for ultrasound diagnosis.

### 2. Description of the Related Art

Ultrasonography for imaging a state of a living body using an ultrasound diagnostic apparatus has been spreading widely in the medical field. In the ultrasound diagnostic apparatus, an ultrasound beam is sent towards the inside of the living body from an array transducer of an ultrasound probe (probe), the array transducer receives ultrasound echoes from inside the living body, the received signal is electrically processed by a main body of the ultrasound diagnostic apparatus, and therefore a state of the living body is imaged. In a case of performing ultrasonography, a coating-type contact medium, called "an ultrasound gel", which has specific acoustic impedance close to the living body is used such that ultrasound waves propagate efficiently between the ultrasound probe and a contact surface of the living body which comes into contact with this ultrasound probe. As performance of the ultrasound gel in addition to the specific acoustic impedance close to the living body, a gel that is difficult to dry, is highly safe, and has no odor has been required.

For example, JP2004-237010A discloses a coating-type contact medium in which practical viscosity is maintained and performance of spreadability and a feeling at the time of coating is enhanced. Furthermore, for example, JP1999-235337A (JP-H11-235337A) discloses a coating composition for ultrasound diagnosis which is less sticky after use, has an appropriate cosmetic effect, and is not needed to be wiped off from a surface of a diagnostic site.

### SUMMARY OF THE INVENTION

The ultrasound probe (probe) is not changed for each patient. After the probe has been used for a previous patient, the ultrasound gel is wiped off, the probe is washed and then used for the next patient. In a case of such a method of use, it has been pointed out that in a case where microorganisms present on the skin originated from the previous patient are attached to the probe, there is a possibility that even if the probe is washed, microorganisms are not sufficiently removed and remain. Therefore, this is desired to be improved.

An object of the present invention is to provide a coating-type contact medium for ultrasound diagnosis which gives a good feeling when the skin is coated with the medium and has antimicrobial effects.

As a result of extensive research to achieve the object, the inventors of the present invention have found that a coating-type contact medium for ultrasound diagnosis which contains a silver-based antimicrobial agent gives a good feeling when the skin is coated with the medium and has antimicrobial effects, and therefore have completed the present invention.

That is, the present invention is described in (1) to (7) as below.

(1) A coating-type contact medium for ultrasound diagnosis, comprising a silver-based antimicrobial agent.
(2) The coating-type contact medium for ultrasound diagnosis according to (1), further comprising a preservative.
(3) The coating-type contact medium for ultrasound diagnosis according to (1) or (2), further comprising a moisturizer.
(4) The coating-type contact medium for ultrasound diagnosis according to (3), in which the moisturizer includes at least one selected from collagen and hydrolyzed collagen.
(5) The coating-type contact medium for ultrasound diagnosis according to any one of (1) to (4), in which the silver-based antimicrobial agent is at least one selected from the group consisting of silver oxide, ceramic-supported silver, and glass-supported silver.
(6) The coating-type contact medium for ultrasound diagnosis according to any one of (1) to (5), further comprising at least one compound selected from the group consisting of a viscosity improver and a lubricant.
(7) The coating-type contact medium for ultrasound diagnosis according to any one of (1) to (6), in which a content of the silver-based antimicrobial agent is 0.004 ppm or more to 1 ppm or less in terms of a mass concentration of silver.

According to the present invention, it is possible to provide a coating-type contact medium for ultrasound diagnosis which gives a good feeling when the skin is coated therewith and has antimicrobial effects.

### DESCRIPTION OF THE PREFERRED EMBODIMENTS

### [Coating-Type Contact Medium for Ultrasound Diagnosis]

The coating-type contact medium for ultrasound diagnosis of the present invention is characterized to contain a silver-based antimicrobial agent.

In a coating-type contact medium for ultrasound diagnosis (commonly known as an "ultrasound gel") of the related art, parabens having a broad antimicrobial spectrum (generally referred to as parahydroxybenzoate) are frequently used as a component having antimicrobial activity. Since parabens are irritative to the skin, restriction values are added to a blending amount thereof in cosmetics and the like. Therefore, a sufficient amount by which antimicrobial effects are sufficiently exhibited cannot be blended, and only an amount by which the bacteriostatic effect is represented can be blended. By blending the silver-based antimicrobial agent in the coating-type contact medium for ultrasound diagnosis, the present invention succeeded to exhibit antimicrobial effects which could not be obtained in a case of using only an antimicrobial agent such as parabens.

First, a component of the coating-type contact medium for ultrasound diagnosis of the present invention (hereinafter will simply be referred to as "the coating-type contact medium of the present invention" in some cases) will be explained.

### 1. Silver-Based Antimicrobial Agent

The coating-type contact medium of the present invention contains a silver-based antimicrobial agent.

In general, examples of the silver-based antimicrobial agent include an agent in which silver is contained in an inorganic antimicrobial agent such as a colloidal silver particles antimicrobial agent, a zeolite-based antimicrobial agent (silver zeolite, silver-zinc zeolite, silver-exchanged zeolite, and the like), a silica gel-based antimicrobial agent (complex silver-silica gel and the like), a glass-based antimicrobial agent (silver ion dispersed-type soluble glass and the like), a silicate-based antimicrobial agent (silver calcium silicate, silver magnesium aluminosilicate, and the like), a titanium oxide-based antimicrobial agent (silver-titanium oxide, silver potassium titanate, and the like), a ceramic-based antimicrobial agent, and a whisker-based antimicrobial agent, an inorganic/organic hybrid antimicrobial agent, and the like.

A preferable silver-based antimicrobial agent among the above is a silver-based antimicrobial substance which is in a particle form and substantially insoluble in water, and examples of a preferable form thereof include at least one selected from the group consisting of silver oxide and supported silver. For example, the agent is selected from the silver-based antimicrobial agent in the form of highly porous micro silver, silver nanoparticles, silver zeolite, and silver glass. Among these, silver oxide, ceramic-supported silver, and glass-supported silver are further preferable.

As such a material representing antimicrobial effects, silver ion water is preferable, and examples thereof include SILVION P (manufactured by JAPAN ION Corporation.), Ag-P WATER (manufactured by SHIN-EI CHEMICAL CO., LTD.), and the like.

A content of the silver-based antimicrobial agent in the coating-type contact medium of the present invention is not particularly limited. Typically, in the coating-type contact medium of the present invention, the silver-based antimicrobial agent is preferably contained within a range of 0.004 ppm to 1.0 ppm, is more preferably contained within a range of 0.1 ppm to 0.9 ppm, and is further preferably contained within a range of 0.2 ppm to 0.8 ppm in terms of a total concentration of silver (in terms of a mass concentration of silver).

If the content of the silver-based antimicrobial agent is 0.004 ppm or more in terms of a total concentration of silver, antimicrobial effects are more favorably exhibited. In addition, regarding antimicrobial effects, an upper limit of the content of the silver-based antimicrobial agent is not particularly limited, but because silver is a precious metal, it is preferable that an amount of use as consumables is less, and therefore a range of 1.0 ppm or less by which effective effects are exhibited is preferable.

In the present invention, in a case where a numerical value range is expressed using "to," this numerical value range includes numerical values at both ends of "to." For example, in a case of expressing "1 to 10", this numerical value range includes 1 and 10.

In order to allow the antimicrobial performance to be exhibited by the silver-based antimicrobial agent, it is preferable that an organic preservative such as paraben is used in combination in addition to use of the inorganic silver-based antimicrobial agent. The preservative to be used in combination may be used alone or may be used in combination of two or more kinds thereof.

### 2. Preservative

The coating-type contact medium of the present invention may further contain a preservative.

By providing the preservative, excellent antimicrobial effects can be exhibited compared to a case where the preservative is not contained.

The preservative is not particularly limited as long as the preservative increases the antiseptic effects of the coating-type contact medium of the present invention and a known preservative of the related art can be used. Examples of the preservative include benzoic acid, benzoic acid salts, alkyldiaminoethylglycine hydrochloride, photosensitizer, chlorocresol, chlorobutanol, salicylic acid, salicylates, sorbic acid, sorbates, dehydroacetic acid, dehydroacetates, trichloro hydroxydiphenyl ether (also known as triclosan), parahydroxybenzoate (also known as paraben), sodium salt of parahydroxybenzoate, phenoxyethanol, phenol, sodium lauryl diaminoethyl glycine, resorcin, pantothenyl ethylbenzoic acid, isopropyl methyl phenol, cetylpyridinium chloride, benzalkonium chloride, benzethonium chloride, chlorhexidine hydrochloride, orthophenyl phenol, sodium orthophenyl phenol, chlorhexidine gluconic acid, cresol, chloramine T, chloroxylenol, chlorphenesin, chlorhexidine, 1,3-dimethylol-5,5-dimethylhydantoin, alkyl isoquinolinium bromide, thianthol, thymol, trichlorocarbanilide, parachlorphenol, halocarban, hinokitiol, zinc pyrithione, piroctone olamine, iodopropynyl butylcarbamate, polyaminopropyl biguanide, methylisothiazolinone, methylchloroisothiazolinone/methylisothiazolinone solution, N,N"-methylenebis[N'-(3-hydroxymethyl-2,5-dioxo-4-imidazolidinyl)urea], palladium dimethylaminostyryl heptyl methyl thiazolium iodide, and the like. In addition, the preservative is not limited to those that are described as a "preservative," and as long as they function as a preservative and do not interfere with the effects of the present invention, they can be used as a preservative.

The preservative can be used alone or may be used in combination of two or more kinds thereof.

As preservative used in the coating-type contact medium of the present invention, at least one selected from the group consisting of parahydroxybenzoate (also known as paraben), sodium salt of parahydroxybenzoate, phenoxyethanol is preferable, a combination of two or more kinds selected from the above is more preferable, and a combination of parahydroxybenzoate and phenoxyethanol is further preferable.

Any one of alkyl ester and aryl ester can be used as parahydroxybenzoate (also known as paraben). Examples of alkyl ester of parahydroxybenzoate include methyl ester (methyl parahydroxybenzoate), ethyl ester (ethyl parahydroxybenzoate), propyl ester (propyl parahydroxybenzoate), isopropyl ester (isopropyl parahydroxybenzoate), butyl ester (butyl parahydroxybenzoate), isobutyl ester (isobutyl parahydroxybenzoate), and the like. Examples of aryl ester include benzyl ester (benzyl parahydroxybenzoate) and the like.

Parahydroxybenzoate can be used alone or may be used in combination of two or more kinds thereof.

As parahydroxybenzoate (also known as paraben) used in the coating-type contact medium of the present invention, at least one selected from the group consisting of methyl parahydroxybenzoate, ethyl parahydroxybenzoate, propyl parahydroxybenzoate, and isopropyl parahydroxybenzoate is preferable, at least two or more kinds selected from the above are more preferable, and it is further preferable to use a combination of methyl parahydroxybenzoate and ethyl parahydroxybenzoate.

A content of the preservative in a case where the coating-type contact medium of the present invention contains the preservative is not particularly limited. A suitable content of the preservative varies according to the types of the preservative, but it is preferable that the content is within a range by which microorganisms in the coating-type contact medium of the present invention can be prevented from growing and the skin or mucosa is not damaged. For example, a total content of parahydroxybenzoate (also known as paraben) is preferably 1.0 % by mass or less, more preferably within a range of 0.1 % by mass to 1.0 % by mass, further preferably within a range of 0.2 % by mass to 1.0 % by mass. In addition, for example, a content of phenoxyethanol is preferably 1.0 % by mass or less, more preferably within a range of 0.1 % by mass to 1.0 % by mass, and further preferably within a range of 0.3 % by mass to 1.0 % by mass. In a case parahydroxybenzoate and phenoxyethanol are used in combination, each content (concentration) can be independently set.

### 3. Moisturizer

The coating-type contact medium of the present invention may further contain a moisturizer.

By providing the moisturizer, a moisture retention property and a feeling of use of the coating-type contact medium of the present invention can further be improved compared to a case where the moisturizer is not contained.

The moisturizer is not particularly limited as long as the moisturizer can increase the moisture retention effect of the coating-type contact medium of the present invention and a known moisturizer of the related art can be used. Examples of the moisturizer include glycine, L-alanine, DL-alanine, L-phenylalanine, L-arginine, L-leucine, L-isoleucine, DL-threonine, L-tyrosine, L-serine, DL-serine, L-glutamic acid, sodium L-glutamate, L-proline, L-valine, L-aspartic acid, sodium L-aspartate, potassium L-aspartate, magnesium L-aspartate, L-histidine hydrochloride, L-lysine hydrochloride, trimethylglycine, taurine, disodium 5'-guanylate, disodium 5'-inosinate, grape sugar (also known as glucose), maltose, maltitol, D-mannite (also known as D-mannitol), water soluble placenta extract, pullulan, sodium hyaluronate, Bifidobacterium extract, urea, D-sorbitol (also known as sorbit and glucitol), silk powder, hydrolyzed silk (hydrolyzed silk solution, hydrolyzed silk powder), hydrolyzed keratin (hydrolyzed keratin solution, hydrolyzed keratin powder), yeast extract, collagen (water soluble collagen, water soluble collagen solution), hydrolyzed collagen, hydrolyzed eggshell membrane, carboxymethyl chitin (carboxymethyl chitin solution), albumin, lecithin, sodium DL-pyrrolidone carboxylic acid solution, DL-pyrrolidone carboxylic acid, L-pyrrolidone carboxylic acid, polyethylene glycol (polyethylene glycol 200, polyethylene glycol 300, polyethylene glycol 400, polyethylene glycol 600, polyethylene glycol 1000, polyethylene glycol 1500, polyethylene glycol 1540, polyethylene glycol 2000, polyethylene glycol 4000, polyethylene glycol 6000, polyethylene glycol 11000, and polyethylene glycol 20000), polypropylene glycol, and the like. In addition, the moisturizer is not limited to those that are described as a "moisturizer," and as long as they function as a moisturizer and do not interfere with the effects of the present invention, they can be used as a moisturizer.

The moisturizer can be used alone or may be used in combination of two or more kinds thereof.

The coating-type contact medium of the present invention comes into direct contact with the skin or mucosa at the time of ultrasonography, and therefore as the moisturizer contained in the coating-type contact medium of the present invention, a component that is skin or mucosa-friendly (a component that is unlikely to irritate the skin or mucosa) is chosen in consideration of safety.

The coating-type contact medium of the present invention preferably contains at least one selected from the group consisting of collagen and hydrolyzed collagen as a moisturizer and more preferably contains hydrolyzed collagen.

The collagen is not particularly limited and may be various collagen extracts. The collagen extract can be extracted from a collagen-containing raw material by a known method such as acid solubilization, alkali solubilization, neutral salt solubilization, and enzyme solubilization. As the collagen-containing raw material, any material can be used as long as the raw material contains collagen. Examples thereof include skin, scale, bone, cartilage, tendon, or organs of vertebrates (for example, bovine, swine, sardine, shark, and the like), but bone, cartilage, skin, scale, tendon, or placenta are suitably used because of high collagen content. Among these, water soluble collagen is particularly preferable as collagen contained in the coating-type contact medium of the present invention.

In addition, protein hydrolysate, particularly oligopeptide, which is obtained by hydrolyzing natural proteins has a chemical structure similar to natural peptides in the epidermis, and thus is widely used as a moisturizer and the like. Therefore, it is particularly preferable to use the hydrolyzed collagen of which collagen is subjected to hydrolysis.

A content of the moisturizer in a case where the coating-type contact medium of the present invention contains the moisturizer is not particularly limited. A suitable content of the moisturizer varies according to the types of the moisturizer, but it is preferable that the content is within a range by which both a water retention property and ease of handling of the coating-type contact medium of the present invention becomes favorable. For example, a total content of collagen and hydrolyzed collagen is preferably within a range of 0.05 % by mass to 20 % by mass, more preferably within a range of 0.1 % by mass to 10 % by mass, and further preferably within a range of 0.2 % by mass to 5 % by mass. If the content is 0.05 % by mass or more, the water retention effect is sufficiently exhibited, and if the content is 20 % by mass or less, ease of handling is sufficiently favorable.

### 4. Viscosity Improver

The coating-type contact medium of the present invention may contain a viscosity improver in addition to the silver-based antimicrobial agent.

By providing the viscosity improver, the viscosity of the coating-type contact medium of the present invention is enhanced and thus safety is enhanced, and a feeling of use can be further improved compared to a case where the viscosity improver is not contained.

The viscosity improver is not particularly limited as long as the viscosity improver can increase the viscosity of the coating-type contact medium of the present invention and a known viscosity improver of the related art can be used. Examples of the viscosity improver include arabic gum, gellan gum, diutan gum, tamarind gum, mannan, transparent cellulose gel, xanthan gum, guar gum, O-[2-hydroxy-3-(trimethylammonio)propyl] guar gum chloride, locust bean gum, pectin, carrageenan, potassium alginate, calcium alginate, sodium alginate, propylene glycol alginate, carboxymethyl cellulose, carboxymethyl cellulose calcium, carboxymethyl cellulose sodium, hydroxyethyl cellulose, hydroxyethyl cellulose ethyl ether, hydroxypropyl methyl cellulose, hydroxypropyl cellulose, O-[2-hydroxy-3-(trimethylammonio) propyl] hydroxyethyl cellulose chloride (also known as hydroxyethyl cellulose hydroxypropyl trimethyl ammonium chloride ether), polyethylene glycol monolaurate, polyethylene glycol dilaurate, polyethylene glycol monostearate, polyethylene glycol distearate, polyethylene glycol monoisostearate, polyoxyethylene sorbitan monolaurate, polyoxyethylene sorbitan monolaurate (20 E.O.), polyoxyethylene sorbitan isostearate (20 E.O.), polyoxyethylene sorbitan isostearate (3 E.O.), polyethylene glycol-160 sorbitan triisostearate, polyvinyl alcohol, carboxyvinyl polymer, calcium-potassium salt of carboxyvinyl polymer, sodium polyacrylate, polyacrylate-13, alkyl polyacrylate, (alkyl acrylate/dimethicone) copolymer, (alkyl acrylate/octyl acrylamide) copolymer, (sodium acrylate/sodium acryloyldimethyltaurate) copolymer, (hydroxyethyl acrylate/sodium acryloyldimethyltaurate) copolymer, (sodium acrylate/acryloyldimethyltaurine/dimethylacrylamide) crosspolymer, (acrylate/polytrimethylsiloxy methacrylate) copolymer, (acrylate/steareth-20 itaconic acid) copolymer, (acrylate/beheneth-25 acrylate) copolymer, (acrylamide/ammonium acrylate) copolymer, dimethicone crosspolymer, polyacrylate crosspolymer-6, (butyl acrylate/glycol dimethacrylate) crosspolymer, (acrylate/(C10-30) alkyl acrylate) crosspolymer, (diphenyl dimethicone/vinyl diphenyl dimethicone/silsesquioxane) crosspolymer, (diphenyl methylsiloxyphenyl methicone/phenyl silsesquioxane) crosspolymer, (dimethicone/(polyethylene glycol-10/15)) crosspolymer, (vinyl dimethicone/methicone silsesquioxane) crosspolymer, (methyl methacrylate/glycol dimethacrylate) crosspolymer, and the like. In addition, the viscosity improver is not limited to those that are described as a "viscosity improver," and as long as they function as a viscosity improver and do not interfere with the effects of the present invention, they can be used as a viscosity improver.

The viscosity improver can be used alone or may be used in combination of two or more kinds thereof.

As the viscosity improver contained in the coating-type contact medium of the present invention, at least one selected from the group consisting of xanthan gum, (acrylate/(C10-30) alkyl acrylate) crosspolymer, alginate ester, carboxymethyl cellulose, polyvinyl alcohol, and transparent cellulose gel is preferable, a combination of two or more kinds selected from the above is more preferable, and a combination of xanthan gum and (acrylate/(C10-30) alkyl acrylate) crosspolymer is further preferable.

A content of the viscosity improver in a case where the coating-type contact medium of the present invention contains the viscosity improver is not particularly limited. A suitable content of the viscosity improver varies according to the types of the viscosity improver, but it is preferable that the content is within a range by which spreadability and a feeling at the time of coating are enhanced by adjusting the viscosity of the coating-type contact medium of the present invention. For example, a content of xanthan gum is preferably within a range of 0.01 % by mass to 0.50 % by mass, more preferably within a range of 0.01 % by mass to 0.10 % by mass, and further preferably within a range of 0.02 % by mass to 0.08 % by mass. In addition, for example, a content of (acrylate/(C10-30) alkyl acrylate) crosspolymer is preferably within a range of 0.10 % by mass to 5.0 % by mass, more preferably within a range of 0.10 % by mass to 1.0 % by mass, and further preferably within a range of 0.50 % by mass to 1.0 % by mass. In a case where xanthan gum and (acrylate/(C10-30) alkyl acrylate) crosspolymer are used in combination, each content (concentration) can be independently set.

### 5. Lubricant

The coating-type contact medium of the present invention may further contain a lubricant.

By providing the lubricant, the friction between an ultrasound probe and a contact surface of a living body which comes into contact with the ultrasound probe can be further decreased and thus slip is improved, and operability of the ultrasound probe can be further improved compared to a case where the lubricant is not contained.

The lubricant is not particularly limited as long as the lubricant can increase the lubricating effect of the coating-type contact medium of the present invention and a known lubricant of the related art can be used. Examples of the lubricant include glycerin, ethylene glycol, diethylene glycol, triethylene glycol, propylene glycol, dipropylene glycol, 1,3-butylene glycol, and the like. In addition, the lubricant is not limited to those that are described as a "lubricant," and as long as they function as a lubricant and do not interfere with the effects of the present invention, they can be used as a lubricant.

The lubricant can be used alone or may be used in combination of two or more kinds thereof.

As the lubricant contained in the coating-type contact medium of the present invention, at least one selected from the group consisting of glycerin, ethylene glycol, propylene glycol, and 1,3-butylene glycol is preferable, and propylene glycol is more preferable.

A content of the viscosity improver in a case where the coating-type contact medium of the present invention contains the lubricant is not particularly limited. A suitable content of the viscosity improver varies according to the types of the viscosity improver, but typically, 1 % by mass to 20 % by mass is preferable, 5 % by mass to 15 % by mass is more preferable, and 5 % by mass to 10 % by mass is further preferable.

The coating-type contact medium of the present invention preferably contains at least one compound selected from the group consisting of the viscosity improver and the lubricant.

### 6. Other Components

In the coating-type contact medium of the present invention, arbitrary components generally used in cosmetics, pharmaceutical external preparations, and the like may be contained in addition to water and the above components within a range by which the effects of the present invention are not impaired. Examples of such arbitrary components include hydrocarbons such as petroleum jelly and microcrystalline wax, esters such as jojoba oil and spermaceti, triglycerides such as beef tallow and olive oil, higher alcohols such as cetanol and oleyl alcohol, fatty acids such as stearic acid and oleic acid, a nonionic surfactant, an anionic surfactant, a cationic surfactant, an amphoteric surfactant, ethanol, a viscosity improver, a preservative, an ultraviolet absorbent, an antioxidant agent, a chelating agent, a coloring agent, a powder, and the like.

### [Method for Manufacturing Coating-Type Contact Medium for Ultrasound Diagnosis]

The coating-type contact medium of the present invention can be produced by dissolving each component described above in water. Heating and stirring may be performed appropriately. In addition, the order of dissolving each component is not particularly limited.

For example, in a case where the coating-type contact medium of the present invention is produced by dissolving silver ion water (silver-based antimicrobial agent), paraben (preservative), phenoxyethanol (preservative), hydrolyzed collagen (moisturizer), (acrylate/(C10-30) alkyl acrylate) crosspolymer (viscosity improver), xanthan gum (viscosity improver), and propylene glycol (lubricant) in water, it is preferable that silver ion water, paraben, phenoxyethanol, hydrolyzed collagen, xanthan gum, and propylene glycol are gradually added to water while stirring, and then heated to be dissolved, and thereafter, (acrylate/(C10-30) alkyl acrylate) crosspolymer is gradually added to the solution while stirring, and then heated to be dissolved.

### [Method of Use Coating-Type Contact Medium for Ultrasound Diagnosis]

The coating-type contact medium of the present invention can be used in the same manner as that of a coating-type contact medium for ultrasound diagnosis of the related art. It is one of the advantageous characteristics of the coating-type contact medium of the present invention that no particular change is required in a method of use.

### [Examples]

### [Example 1]

### (1) Preparation of Coating-Type Contact Medium

XANTHAN GUM (manufactured by SANSHO Co., Ltd.), PROPYLENE GLYCOL (manufactured by Asahi Glass Co., Ltd.), METHYL PARAHYDROXYBENZOATE (manufactured by Wako Pure Chemical Industries, Ltd.), ETHYL PARAHYDROXYBENZOATE (manufactured by Wako Pure Chemical Industries, Ltd.), PHENOXYETHANOL (manufactured by Yokkaichi Chemical Company, Limited.), HYDROLYZED COLLAGEN (manufactured by SEIWA KASEI Co. Ltd.), and SILVER ION WATER (manufactured by SHIN-EI CHEMICAL CO., LTD.; (trade name) Ag-P WATER; (component) silver oxide, borosilicate (Ca/Na), water; total silver concentration = about 4.0 ppm (mass concentration converted into silver by atomic absorption spectroscopy)) were prepared and weighed such that a final concentration became as shown in Table 1, were gradually added to purified water while stirring, and then heated to be dissolved. To the solution obtained by completely dissolving each of the above components, (ACRYLATE/(C10-30) ALKYL ACRYLATE) CROSSPOLYMER (manufactured by Lubrizol Advanced Materials) weighed such that a final concentration became as shown in Table 1 was gradually added while stirring so as to be dissolved, and therefore a coating-type contact medium for ultrasound diagnosis (ultrasound gel) was prepared.

### (2) Evaluation of Coating-Type Contact Medium

Evaluation of the prepared coating-type contact medium was performed by the following method.

### 2.1) Evaluation on Feeling

The abdomen of five subjects was coated with 3 g of the coating-type contact medium, and five subjects answered to which of the following standards the feeling belonged. The majority of the answers were evaluated. The results of the evaluation are shown in Table 1.
A: Moisturization is sufficiently effective, and there is no discomfort
B: Moisturization is insufficient, and there is discomfort of a dry feeling

### 2.2) Evaluation on Antimicrobial Property

### 2.2.1)

### (Test Sample)

0.01 mL of bacterial solution was added to 1 g of the coating-type contact medium and mixed, and the mixture was left to stand at room temperature of 25°C ± 3°C for a predetermined time. After being left to stand, 9 mL of an SCDLP (Soybean-Casein Digest Agar with Lecithin & Polysorbate 80) medium was added thereto and mixed to inactivate the antimicrobial activity of the coating-type contact medium, and therefore the number of living bacteria was measured.

### (Reference Sample)

0.01 mL of bacterial solution was added to 1 mL of purified water and mixed, and the mixture was left to stand under the same conditions as the test sample. After being left to stand, 9 mL of the SCDLP medium was added thereto and mixed, and therefore the number of living bacteria was measured.

### 2.2.2)

At the lapse of 5 minutes, the test sample and the reference sample were compared to evaluate the antimicrobial property according to the following evaluation standards. The evaluation results are shown in Table 1.

### (Evaluation Standard)

A: At the lapse of 5 minutes after the bacterial solution was added to the test sample and mixed with a mixer, the number of living bacteria decreased greatly with respect to the number of bacteria in the reference sample
B: Decrease of bacteria is slightly inferior to A but is practically acceptable
C: Decrease of bacteria is small and is not practically acceptable
D: No decrease of bacteria is shown

### [Examples 2 to 4]

A coating-type contact medium was prepared in the same manner as in Example 1 except that an addition amount of silver ion water was changed such that a final concentration became as shown in Table 1. Evaluation on "the feeling" and "the antimicrobial property" was carried out in the same manner as in Example 1, using the prepared coating-type contact medium. The evaluation results are shown in Table 1.

### [Examples 5 and 6]

A coating-type contact medium was prepared in the same manner as in Example 1 except that an addition amount of silver ion water was changed such that a final concentration became as shown in Table 1, and that none of methyl parahydroxybenzoate, ethyl parahydroxybenzoate, or a phenoxyethanol preservative was added. Evaluation on "the feeling" and "the antimicrobial property" was carried out in the same manner as in Example 1, using the prepared coating-type contact medium. The evaluation results are shown in Table 1.

### [Comparative Example 1]

A coating-type contact medium was prepared in the same manner as in Example 1 except that none of silver ion water, methyl parahydroxybenzoate, ethyl parahydroxybenzoate, or phenoxyethanol was added. Evaluation on "the feeling" and "the antimicrobial property" was carried out in the same manner as in Example 1, using the prepared coating-type contact medium. The evaluation results are shown in Table 1.

### [Comparative Example 2]

A coating-type contact medium was prepared in the same manner as in Example 1 except that silver ion water was not added. Evaluation on "the feeling" and "the antimicrobial property" was carried out in the same manner as in Example 1, using the prepared coating-type contact medium. The evaluation results are shown in Table 1.

**[Table 1]**

| | | | | Example | | | | | | Comparative Example | |
|---|---|---|---|---|---|---|---|---|---|---|---|
| | | | | 1 | 2 | 3 | 4 | 5 | 6 | 1 | 2 |
| Component | Silver-basted antimicrobial agent | Silver ion water (total silver concentration) | [ppm] | 0.04 | 0.2 | 0.4 | 0.8 | 0.4 | 0.8 | - | - |
| | Preservative | Methyl parahydroxybenzoate | [% by mass] | 0.2 | 0.2 | 0.2 | 0.2 | - | - | - | 0.2 |
| | | Ethyl parahydroxybenzoate | [% by mass) | 0.05 | 0.05 | 0.05 | 0.05 | - | - | - | 0.05 |
| | | Phenoxyethanol | [% by mass] | 0.3 | 0.3 | 0.3 | 0.3 | - | - | - | 0.3 |
| | Moisturizer | Hydrolyzed collagen | [% by mass] | 1 | 1 | 1 | 1 | 1 | 1 | 1 | 1 |
| | Viscosity improver | (Acrylate/(C10-30) alkyl acrylate) crosspolymer | [% by mass] | 0.65 | 0.65 | 0.65 | 0.65 | 0.65 | 0.65 | 0.65 | 0.65 |
| | | Xanthan gum | [by % mass] | 0.05 | 0.05 | 0.05 | 0.05 | 0.05 | 0.05 | 0.05 | 0.05 |
| | Lubricant | Propylene glycol | [% by mass] | 8 | 8 | 8 | 8 | 8 | 8 | 8 | 8 |
| | Water | Purified water | | Residue | Residue | Residue | Residue | Residue | Residue | Residue | Residue |
| Evaluation | Feeling (2 stages of A and B) | | | A | A | A | A | A | A | A | A |
| | Antimicrobial property (4 stages of A, B, C, and D) | | | A | A | A | A | B | B | D | C |

From the evaluation results shown in Table 1, it was confirmed that the coating-type contact medium containing the silver-based antimicrobial agent (Examples 1 to 6) gave a good feeling when the skin was coated therewith and had antimicrobial effects.

In addition, according to the comparison between Examples 3 and 4 and Examples 5 and 6, in a case of using the silver-based antimicrobial agent and the preservative in combination, the evaluation on the antimicrobial property was good and excellent antimicrobial property was checked compared to a case where the preservative was not used in combination.

## Claims

1. A coating-type contact medium for ultrasound diagnosis, comprising:
a silver-based antimicrobial agent.

2. The coating-type contact medium for ultrasound diagnosis according to claim 1, further comprising:
a preservative.

3. The coating-type contact medium for ultrasound diagnosis according to claim 1 or 2, further comprising:
a moisturizer.

4. The coating-type contact medium for ultrasound diagnosis according to claim 3,
wherein the moisturizer includes at least one selected from collagen and hydrolyzed collagen.

5. The coating-type contact medium for ultrasound diagnosis according to any one of claims 1 to 4,
wherein the silver-based antimicrobial agent is at least one selected from the group consisting of silver oxide, ceramic-supported silver, and glass-supported silver.

6. The coating-type contact medium for ultrasound diagnosis according to any one of claims 1 to 5, further comprising:
at least one compound selected from the group consisting of a viscosity improver and a lubricant.

7. The coating-type contact medium for ultrasound diagnosis according to any one of claims 1 to 6,
wherein a content of the silver-based antimicrobial agent is 0.004 ppm or more and 1 ppm or less in terms of a mass concentration of silver.
